# EUROPEAN PATENT APPLICATION

(11) **EP 1 992 702 A1**
(43) Date of publication of application: **19.11.2008**
(21) Application number: 07108173.1
(22) Date of filing: 14.05.2007
(51) Int. Cl.: C12Q 1/68

(54) **Detection methods with increased accuracy and/or sensitivity**

(71) Applicant: Koninklijke Philips Electronics N.V., 5621 BA Eindhoven (NL)
(72) Inventor: The designation of the inventor has not yet been filed
(74) Representative: Van Velzen, Maaike Mathilde

(57) **Abstract**

Methods for increasing the sensitivity and precision of analyte detection are provided, using multiple optically active labels for identification of analytes.

## Description

### FIELD OF THE INVENTION

The present invention relates to methods, kits and tools for the detection and/or quantification of analytes with improved sensitivity and/or accuracy.

### BACKGROUND TO THE INVENTION

The sensitive and accurate detection, either qualitatively or quantitatively, of low concentrations of biomolecules such as proteins, peptides, oligonucleotides, nucleic acids, lipids, polysaccharides, hormones, neurotransmitters, metabolites, etc... has proven to be an elusive goal with widespread potential uses in medical diagnostics, pathology, toxicology, epidemiology, biological warfare, environmental sampling, forensics and numerous other fields such as comparative proteomics and gene expression studies.

A particular example is the detection of DNA in, e.g., medical diagnostics (detection of infectious agents such as pathogenic bacteria and viruses, diagnosis of inherited and acquired genetic diseases, etc...), in forensic tests as part of criminal investigations, in paternity disputes, in whole genome sequencing, etc...

While the identification and/or quantification of a purified sample of a biological analyte can sometimes be performed based on the physicochemical properties of the analyte itself, most detection methods, which are capable of identifying and/or quantifying an analyte in a non-purified sample, make use of a "probe" which is a known molecule having a strong affinity and preferably also a high degree of specificity for the analyte. Where the analyte is a protein or peptide, these assays are referred to as ligand-binding assays (e.g., immunoassays). Detection of DNA typically makes use of the hybridization of a nucleotide sequence that is specific to the target DNA.

In these probe-based detection assays the analyte-specific probe (or the analyte itself) is either directly or indirectly labelled with a traceable substance. The detection of the traceable substance (hereafter referred to as "label") is indicative of the amount of analyte in the test sample. Detection of the label can be ensured using a variety of different techniques, depending upon the nature of the label employed.

Current detection methods typically involve detection of fluorescently labelled antibodies or oligonucleotide probes that can bind to the analyte or target biomolecule. Cross-reactivity and non-specific binding may complicate fluorescent detection of biomolecules in complex samples. Even where high probe-specificity is obtained, the sensitivity of fluorescent detection is often insufficient to identify low concentrations ofbiomolecules. Fluorescence spectra contain broad bands typically in the order of 40-60 nanometer: therefore to detect multiple fluorescent species in solution one needs to use multiple excitation wavelengths to discriminate between the broad band spectra.

Surface enhanced (resonance) Raman spectroscopy or SE(R)RS is a technique that is rapidly gaining on fluorescence for detection in view of its impressive sensitivity. Raman spectroscopy utilizes the phenomenon of Raman scattering. When light passes through an optically transparent sample, a fraction of the light is scattered in all directions. Most of the scattered photons are of the same wavelength as the incident light. This is known as Rayleigh scattering. However, a small fraction of the scattered light has a different wavelength and a slight random alteration in phase. The wavelengths of the Stokes (Anti-Stokes) Raman emission spectrum are shifted to longer (or shorter) wavelengths relative to the excitation wavelength. The Raman spectrum is characteristic of the chemical composition and structure of the molecules in a sample with very narrow emission bands, while the intensity of Raman scattering is linearly dependent on the concentration of these molecules. The intrinsically weak Raman scattering can be enhanced by factors of up to 10⁸ or more when a compound is adsorbed on or near roughened metal surfaces, e.g., nanoparticles, of gold, silver, copper and certain other metals. The technique associated with this phenomenon is known as surface-enhanced Raman scattering (SERS). The increase in detection sensitivity is more marked the closer the analyte sits to the "active" surface. The optimum position is in the first molecular layer around the surface, i.e., within about 30 nm of the surface.

A further 10³ fold increase in sensitivity can be obtained by operating at the resonance frequency of the analyte or, as is more commonly done, making use of a 'SERS-active' substance or dye attached to the analyte (capable of generating a SE(R)RS spectrum when appropriately illuminated), and operating at the resonance frequency of the dye. This is termed "resonance Raman scattering" spectroscopy. The combination of the surface enhancement effect and the resonance effect to give "surface enhanced resonance Raman scattering" or SE(R)RS strongly increases the sensitivity. When compared to fluorescence, a SE(R)RS signal can be more easily discriminated from contamination and background due to its sharp vibrational bands instead of broad electronic bands in fluorescence.

Another key advantage of SE(R)RS is the possibility of multiplexing using a single excitation wavelength. Each SE(R)RS-active dye used as a label gives a unique fingerprint which can be recognized in a dye mixture without separation as would be necessary for fluorescence spectroscopy. There are many dyes, either specially designed dyes for SE(R)RS or dyes that were previously used in other detection methods, each with a unique spectrum. SE(R)RS is thus a highly sensitive and specific method for biomolecule detection giving increased sensitivity for the detection of low concentrations of biomolecules. Such multiplexing methods have been described in, e.g., W02005/066373. Bioanalytical techniques using SE(R)RS have been demonstrated to allow detection of attomole (10⁻¹⁸ mol) quantities of proteins or DNAs down to femtomole (10⁻¹⁵ mol in 400 µl) concentrations. Single-molecule detection limits have been reported for rhodamine 6G (R6G), adenine, crystal violet, and other SE(R)RS-active molecules. Raman spectroscopy is applied very broadly, from material analysis in physics to a very wide variety of applications in biology.

Optimisation of SE(R)RS-based detection methods has typically focused on improving the quantitative detection of analytes (Faulds et al., Anal. Chem., 2004, 76: 412-417). More recent studies have described conditions for the optimisation of reproducibility of SE(R)RS-based measurements (W02007023452). There is still however a need for yet faster and more accurate SE(R)RS-based detection methods in order to accommodate the increasingly growing fields of diagnosis or DNA array, for instance.

### SUMMARY OF THE INVENTION

It is an object of the present invention to provide methods, kits and tools for the detection and/or quantification of analytes.

An advantage of the present invention is improved sensitivity and/or accuracy, e.g. an increase in the detectable signal generated by an analyte thereby further increasing the sensitivity of optical detection methods. Accordingly, the present invention teaches the use of multiple labels to detect and/or quantify an analyte in a sample.

It is also an advantage of the present invention to provide a control on the accuracy of analyte detection based on the detection of a signal generated by the binding of a label to an analyte and therefore to increase the reliability of optical detection methods. Accordingly, the present invention teaches the incorporation of two or more different optical labels to detect and/or quantify an analyte in a sample.

Particular and preferred aspects of the invention are set out in the accompanying independent and dependent claims. Features from the dependent claims may be combined with features of the independent claims and with features of other dependent claims as appropriate and not merely as explicitly set out in the claims.

A first aspect of the invention provides methods of detection of one or more analytes in a sample, more particularly methods with increased sensitivity, comprising the steps of providing, for each analyte, on a nucleic acid molecule, which is the analyte, a fragment thereof, or a nucleic acid sequence linked to the analyte, two or more labels having the same optical properties and detecting the optical spectrum of said labels provided on said nucleic acid molecule as a measure for the amount of analyte in the sample.

In a particular embodiment, the methods of the invention encompass amplification of the nucleic acid molecule, which is the analyte itself, a fragment thereof, or a nucleic acid sequence which has been linked to the analyte. Accordingly, the present invention provides methods of detection of one or more analytes in a sample, comprising the steps of amplifying, for each analyte, a nucleic acid molecule which is the analyte, a fragment thereof or a nucleic acid sequence linked to the analyte in a nucleic acid amplification process, and providing on the amplified nucleic acid molecule two or more labels having the same optical properties. These methods further comprise the step of detecting the optical spectrum of the labels provided on the amplified nucleic acid molecule, whereby the cumulative detection of the spectrum of the labels is indicative of the presence and/or quantity of analyte in the sample.

Methods of the present invention are particularly suited for use with SE(R)RS labels or fluorescent labels, whereby the presence of more than one label per analyte molecule increases sensitivity of detection.

In particular embodiments of the invention, the methods are directed to the detection of an analyte which is a nucleic acid and the nucleic acid amplification process is performed on the analyte or a fragment thereof.

In alternative embodiments, the analyte is a molecule which is not a nucleic acid that can be subjected to a nucleic acid amplification process, and the method comprises the step of linking a nucleic acid molecule to the analyte. More particularly, the method comprises the step of detecting the analyte with an analyte-specific capture molecule, such as an antibody, whereby the analyte-specific capture molecule is/can be linked to a nucleic acid molecule.

In particular embodiments, methods of the present invention comprise the step of amplifying a nucleic acid molecule (corresponding to either the analyte, a fragment thereof or a nucleic acid linked to the analyte) using primers each of which comprises one or more labels having the same optical properties. In further particular embodiments, primers are used each of which comprises one or more SE(R)RS labels having identical optical properties and the length of the fragment of the nucleic acid amplified during the nucleic acid amplification process is optimised for gain in sensitivity of SE(R)RS. Where the labels used are fluorescence labels the length of the amplified nucleic acid is optimised to avoid quenching of the fluorescent labels.

In further particular embodiments, methods according to this aspect of the invention comprise amplifying the nucleic acid molecule, corresponding to the analyte, a fragment thereof or a nucleic acid linked to the analyte, using nucleotides of which at least a percentage comprise a label.

In particular embodiments, the nucleotides comprise SE(R)RS labels and the methods of the invention comprise the optimization of the percentage of labelled nucleotides used in the nucleic acid amplification process for gain in sensitivity of SE(R)RS.

In further embodiments, methods of the present invention comprise amplifying a nucleic acid molecule, corresponding to the analyte, a fragment thereof or a nucleic acid linked to the analyte, and allowing the amplified nucleic acid molecules to hybridize to one or more nucleic acid probes, whereby the one or more nucleic acid probes together comprise at least two labels having the same optical properties. More particularly, in one embodiment, each nucleic acid probe comprises one label. Alternatively, in another embodiment, each nucleic acid probe comprises more than one label.

In particular embodiments of the invention, the methods envisage PCR, RCA, NASBA, or linear amplification as the nucleic acid amplification process used to amplify nucleic acid, corresponding either to the analyte, a fragment thereof or a nucleic acid sequence linked to the analyte. The resulting amplified nucleic acids carry two or more labels having the same optical properties.

In particular embodiments, methods of the invention comprise the step of correlating the intensity of the detected spectrum, e.g. the SE(R)RS spectrum of the labels provided on the amplified nucleic acids to the amount of analyte in the sample.

A second aspect of the invention provides methods of detection of one or more analytes in a sample with increased precision, comprising the steps of providing on a nucleic acid molecule, which is the analyte, a fragment thereof, or a nucleic acid sequence linked to the analyte, two or more labels having different optical properties and detecting the spectrum of each of said labels provided on said nucleic acid molecule. This allows the detection of different signals, each of which is representative of the analyte in the sample.

According to particular embodiments, methods of the present invention encompass the amplification of a nucleic acid molecule, which is the analyte itself, a fragment thereof or a nucleic acid sequence linked to the analyte. Accordingly, the present invention provides methods for the detection of one or more analytes in a sample comprising the step of amplifying, for each analyte, a nucleic acid molecule, which is the analyte, a fragment thereof or a nucleic acid sequence linked to the analyte, in a nucleic acid amplification process, thereby ensuring that two or more optically active labels having different optical properties are provided on the amplified nucleic acid molecule. The methods according to this aspect of the invention further comprise the step of detecting, for each analyte, the spectrum of each of the labels provided on the amplified nucleic acid molecule, whereby the detection of the spectrum of each of the different labels is indicative of the presence and/or quantity of the analyte in the sample and comparison of the presence and/or intensity of the spectrum of the different labels provided on the amplified nucleic acid is indicative of the accuracy of detection.

Methods according to this aspect of the present invention are particularly suited for use with SE(R)RS labels or fluorescent labels, whereby the detection of different labels per analyte molecule increases accuracy of detection.

In particular embodiments, methods according to the above aspect of the present invention comprise the step of amplifying a nucleic acid molecule (corresponding to either the analyte, a fragment thereof or a nucleic acid linked to the analyte) using primers which comprises at least two SE(R)RS labels having different optical properties. This ensures that different optical labels are provided on the amplified nucleic acid molecule.

In further particular embodiments, methods according to this aspect of the invention comprise amplifying the nucleic acid molecules using nucleotides comprising at least two labels having different optical properties. Again, this ensures that different optical labels are provided on the amplified nucleic acid molecule.

Further embodiments according to this aspect of the present invention encompass methods which comprise amplifying a nucleic acid molecule, which is the analyte, a fragment thereof or a nucleic acid molecule linked to the analyte, and allowing the amplified nucleic acid molecule to hybridise with two or more nucleic acid probes, each of the nucleic acid probes being complementary to a different sequence of the amplified nucleic acid molecule and each probe comprising at least one label having different optical properties. This is yet another way to ensure that different optical labels are provided on the amplified nucleic acid molecule.

In certain embodiments of the invention, methods are used to detect analytes which are not nucleic acid molecules. These embodiments of the invention comprise an additional step of linking a nucleic acid molecule to the analyte through an analyte capture molecule. In particular embodiments, this non-nucleic acid molecule is a peptide, a polypeptide or a protein. According to further particular embodiments, the non-nucleic acid molecule is linked to a nucleic acid molecule directly or indirectly through an antibody.

Particular embodiments, however, of the above described methods of the present invention relate to detection of analytes which are polynucleotides, such as DNA, RNA, or PNA.

In further particular embodiments of the methods of the invention, the nucleic acid amplification process used to amplify the nucleic acid, which corresponds either to the analyte, a fragment thereof or a nucleic acid sequence linked to the analyte, is PCR, RCA, NASBA, or linear amplification.

A third aspect of the invention provides kits for use in the detection of an analyte in a sample. In particular embodiment, the kits comprise at least three primers or probes specific for the analyte to be detected, each primer carrying two or more optically active labels. Alternatively, the kits according to the present invention comprise at least three primers or probes specific for the analyte to be detected, each of which comprising one or more optically active labels.

According to particular embodiments of this aspect of the present invention, the optically active labels provided in the kits are SE(R)RS labels having the same optical properties. In yet another embodiment of the kits of the present invention, the optically active labels are SE(R)RS labels having different optical properties.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### BRIEF DESCRIPTION OF THE FIGURES

FIG. 1 is a schematic drawing showing a double stranded DNA obtained after amplification of a target DNA using primers containing multiple labels in accordance with an embodiment of the present invention. The labels present on the primers can be identical or different.
FIG. 2 is a schematic drawing showing a double stranded DNA obtained after amplification of a target DNA using labelled nucleotides in accordance with an embodiment of the present invention. One or more labelled nucleotide can be used in the amplification step.
FIG. 3 A. is a schematic drawing showing a double stranded DNA obtained after hybridization onto a target DNA of multiple SE(R)RS-active complementary probes carrying the same label in accordance with an embodiment of the present invention.
FIG. 3 B. represents the corresponding resulting SE(R)RS spectra. The signal strength is increased by the use of multiple labels (plain line) in accordance with an embodiment of the present invention, which leads to a more sensitive detection, compared to a single label (dotted line).
FIG. 4 A. is a schematic drawing showing a double stranded DNA obtained after hybridization onto a target DNA of multiple SE(R)RS-active complementary probes carrying different labels in accordance with an embodiment of the present invention.
FIG. 4 B. represents the corresponding resulting SE(R)RS spectra. The combination of the different spectra enables a higher precision of the measurement by introducing an internal control in accordance with an embodiment of the present invention.

### DETAILED DESCRIPTION

The following terms or definitions are provided solely to aid in the understanding of the invention. Unless specifically indicated, these definitions should not be construed to have a scope less than understood by a person of ordinary skill in the art. Where the term "comprising" is used in the present description and claims, it does not exclude other elements or steps. Where an indefinite or definite article is used when referring to a singular noun, e.g., "a" or "an", "the", this includes a plural of that noun unless something else is specifically stated.

The term "analyte", as used herein, refers to the substance to be detected and/or quantified in the methods of the present invention. A non-limiting list of analytes envisaged by the present invention is provided herein.

The term "label", as used herein, refers to a molecule or material capable of generating a detectable signal. The term optical label or label with optical properties refers to a label that can be detected by optical detection methods such as a SE(R)RS label or a fluorescent label. The term 'SE(R)RS label or dye' is used to refer to a label which is capable of generating a SE(R)RS spectrum when appropriately illuminated. A non-limiting list of labels envisaged for use in the methods of the present invention is provided below. Two or more (SE(R)RS) labels with identical optical properties are either identical (SE(R)RS) labels or (SE(R)RS) labels of which the (SE(R)RS) spectrum can not be distinguished. It is understood to the skilled person that similar molecules may be used either as fluorescence or as SE(R)RS labels.

The term "analyte-specific probe" as used herein, refers to a probe comprising a structure or sequence which is specific for the analyte to be detected and which can thus bind specifically to the analyte. The binding of the analyte-specific probe to the analyte can be based on any type of interaction including but not limited to complementary nucleotide sequences, antigen/antibody interaction, ligand/receptor binding, enzyme/substrate interaction, etc...

The term 'amplified nucleic acid' or 'amplicon' as used herein refers to the product of a nucleic acid amplification reaction. Where the analyte is a nucleic acid and the amplification reaction is performed directly on the analyte, the amplified nucleic acid can correspond to the analyte or a fragment thereof. Alternatively, where the amplification reaction is performed on a nucleic acid which is not the analyte (but bound directly or indirectly to the analyte), the amplified nucleic acid can be different from the analyte.

A "sample" as used herein relates to a composition believed to comprise at least one analyte of interest.

A "control sample" as used herein relates to a composition that is identical or very similar to the sample which is generally used to check the accuracy of detection.

A "SE(R)RS active surface" as used herein refers to a material which is capable of enhancing the signal of a SE(R)RS label.

Methods of the present invention are based on the observation that it is possible to further increase the sensitivity of detection methods, most particularly optical detection methods in a simple and easily applicable manner, thereby potentially reducing the time of sample preparation and hence the overall analysis time. The same principle also makes it possible to increase the accuracy of optical detection by by the detection of two or more different labels to determine the presence of the same analyte in a sample.

Accordingly, the present invention provides detection methods, more particularly methods using optically active labels such as SE(R)RS labels wherein, by making use of multiple labelling, a more sensitive and reliable detection and/or quantification of an analyte in a sample is obtained. In particular embodiments of the invention multiple labelling is combined with nucleic acid amplification, to further increase sensitivity of detection.

The most straightforward embodiments of the methods of the invention involve the detection of one or more analytes in a sample by providing the analyte with two or more labels having identical optical properties to increase the signal. This is ensured by making use of either one multiply labelled probe or by contacting the analyte with two or more probes each carrying at least one optical label. Where the analyte of interest is a nucleic acid molecule, the labelled probes are each directed against a different sequence within the analyte, such that the different probes can bind to the analyte simultaneously. The sequences targeted by the analyte-specific probes can be selected such that the cumulative signal generated upon the binding of the different probes to the analyte is optimal. More particularly, where the labels are SE(R)RS labels or fluorescent labels, the position of the probes on the analyte is optimized for optimal SE(R)RS/fluorescent detection. Where the analyte is a protein or peptide, the use of multiple labeled probes is also envisaged. For instance, different antibodies (or antigen-binding fragments thereof) each binding to a different epitope of the analyte, but labelled with a label having the same optical properties, are used simultaneously to ensure amplification of the detection signal.

In particular embodiments, methods of the invention involve nucleic acid amplification, as this method allows the generation of multiple copies of a molecule whereby either a specific sequence and/or specific labels can be introduced. Typically, where the analyte to be detected is a nucleic acid molecule, the methods of the present invention involve the amplification in a nucleic acid amplification method of the analyte or a part thereof. The primers used in such an amplification reaction are specific for the analyte, and the amplified nucleic acid corresponds to a sequence specific for the analyte. In alternative embodiments of the present invention however, the amplification reaction is performed on a nucleic acid sequence, which is not the analyte or a fragment thereof, but a sequence linked directly or indirectly to the nucleic acid. For instance, where the analyte is a protein or peptide, an artificial nucleic acid sequence is linked to the analyte and then amplified by immuno-PCR, and the amplified nucleic acid corresponds to the entire artificial nucleic acid sequence or a part thereof. In methods making use of immuno-PCR, the detection of the amplified nucleic acid molecule is indicative of the presence of the analyte in the sample. Accordingly, methods of the present invention are not limited to the detection of nucleic acids which can be amplified as a whole or in part in a nucleic acid amplification reaction. In these alternative embodiments, methods of the invention encompass the amplification of a nucleic acid sequence linked to the analyte. Different methods of linking a nucleic acid to an analyte of interest are envisaged and include methods based on antigen-antibody interactions, sugar-lectin interaction, etc...

According to a first aspect, the present invention provides methods for increasing the sensitivity of optical detection methods, more particularly SE(R)RS-based detection methods of one or more analytes in a sample. This is generally achieved by directly or indirectly labelling (each of) the analyte(s) with two or more labels having identical optical properties, such that the optical signal is increased. Most particularly the methods of the invention comprise the use of more than one labelled probe which either binds the analyte directly or a sequence linked to the analyte.

In particular embodiments, methods of the invention encompass amplifying the analyte or a nucleic acid sequence linked specifically thereto and ensuring that the amplified nucleic acid sequence comprises multiple identical labels, thus increasing the optical signal to be detected.

In a first embodiment, methods of the invention encompass amplifying the analyte, a fragment thereof or a nucleic acid sequence linked thereto and thereby introducing two or more labels with identical optical properties into the amplified nucleic acid sequence.

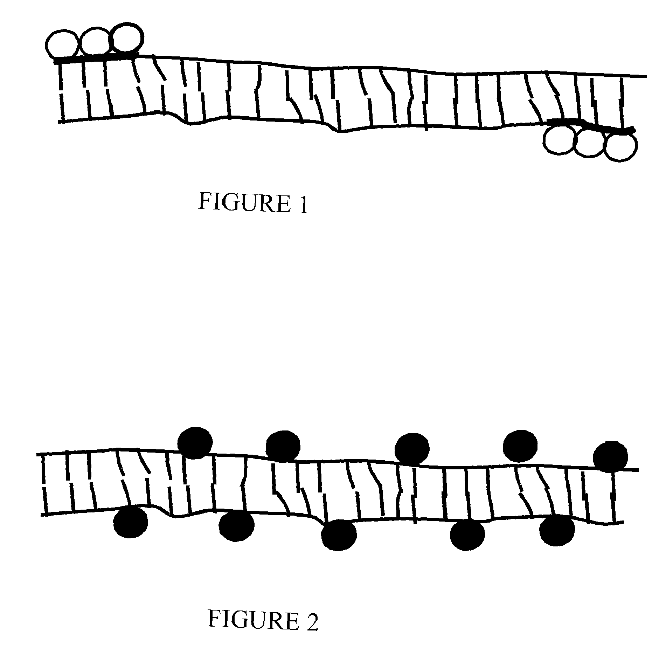

In a particular embodiment, methods of the invention comprise the amplification of the analyte, a fragment thereof or a nucleic acid sequence linked thereto in a nucleic acid amplification method using one primer with two or more labels or two or more primers each comprising one or more, most particularly two or more labels, wherein all labels have the same optical properties. The resulting amplified nucleic acid comprises at least two labels, thereby increasing the strength of the signal generated by the amplified nucleic acid compared to when only one (singly) labelled primer had been used, as is classically the case. The detection of the two or more optical labels incorporated into the amplified nucleic acid molecule is indicative of the presence of the analyte in the sample. A particular embodiment is illustrated in Figure 1. Here two labelled primers are used in the amplification reaction of a nucleic acid molecule, whereby each primer comprises three identical labels.

Methods for generating primers comprising one or more optical labels for use in the methods of the present invention are known to the skilled person. These include, e.g., methods whereby oligonucleotides are labelled after synthesis. The optical label can be provided on the nucleic acid, either as an intercalator or by direct covalent attachment (Faulds et al. (2005) Analyst, 130:1125-1131). Additionally or alternatively, an optical label, such as a SE(R)RS label or a fluorescent label can be chemically added at the 5' end of the nucleic acid, e.g., using the EDAC (1-ethyl-3,3-dimethylaminopropyl carbodiimide) method as described in, e.g., Vo-Dinh et al. (1999) J. Raman Spectro. 30:785-793, and U.S. Patent No. 5,814,516. Further methods of labelling oligonucleotides include the chemical modification of the deoxyribose by replacing a DNA base with a SE(R)RS or fluorescent label. This can be done throughout the sequence, thereby integrating more than one label (Cuppoletti et al. (2005) Bioconjug. Chem. 16(3):528-534). An optical label can also be chemically bound to a number of internal phosphate in the oligonucleotide (Mineno et al. (1993) DNA seq. 4(3):135-141).

Alternatively, optically labelled primers can be generated by making use of labelled nucleotides (see below). SE(R)RS-reactive labels that contain reactive groups designed to covalently react with other molecules, such as nucleotides or nucleic acids, are commercially available (e.g., Molecular Probes, Eugene, OR). SE(R)RS-reactive or fluorescent labels that are covalently attached to nucleotide precursors may be purchased from standard commercial sources (e.g., Roche Molecular Biochemicals, Indianapolis, IN; Promega Corp., Madison, WI; Ambion, Inc., Austin, TX; Amersham Pharmacia Biotech, Piscataway, N.J.). The use of modified nucleotides has successfully been used to synthesize multiply labelled oligonucleotides (Misra et al. (2004) Bioconjug. Chem. 15(3):638-646 and Brumbaugh et al. (1988) PNAS 85:5610-5614).

Optionally, the reaction used to incorporate two or more labels into the amplified nucleic acid is, e.g., a PCR, an exponential circle amplification reaction (RCA), or a nucleic acid sequence-based amplification (NASBA) involving the use of two primers, each directed against a different sequence specific for the analyte or fragment thereof or for the nucleic acid molecule linked thereto. In these embodiments, both primers each comprise two or more optical labels. Alternatively, nucleic acid amplification can be performed using only one primer (e.g., classical linear polymerase amplification or linear rolling circle amplification). In these embodiments the single primer used comprises two or more optical labels, resulting in each amplicon comprising two or more labels with identical optical properties.

According to another embodiment, methods of the invention comprise the generation of a multiply labelled amplicon by amplifying the analyte, a fragment thereof or a nucleic acid molecule linked to the analyte in a nucleic acid amplification step thereby incorporating labelled nucleotides in the amplicon. In particular, at least a fraction of the nucleotides used in the amplification process carry one or more labels compatible with optical detection, such as SE(R)RS-based detection. A single nucleotide, e.g., A, C, T, U, or G, carrying one or more identical optical labels or several different nucleotides carrying one or more labels with the same optical properties can be used in the process, as long as the resulting amplified nucleic acid molecule comprises more than one, most particularly more than two of the same label.

The concentration of labelled nucleotides used during the amplification step is optionally optimized to produce the strongest possible signals with the best signal-to-noise ratio. It is envisaged that any of the four nucleotides is labelled or a percentage of all nucleotides used is labelled or combinations thereof. Suitable percentages include any percentage between 1-100%, depending on the incorporation rate of the nucleotide in the amplified nucleic acid.

The incorporation of labelled nucleotides according the methods of the present invention can similarly be performed using any known method of nucleic acid amplification, such as, but not limited to PCR, RCA, NASBA etc.

SE(R)RS-reactive or fluorescent labels that contain reactive groups designed to covalently react with other molecules, such as nucleotides or nucleic acids, are commercially available (e.g., Molecular Probes, Eugene, OR). SE(R)RS-reactive and fluorescent labels that are covalently attached to nucleotide precursors may be purchased from standard commercial sources (e.g., Roche Molecular Biochemicals, Indianapolis, IN; Promega Corp., Madison, WI; Ambion, Inc., Austin, TX; Amersham Pharmacia Biotech, Piscataway, N.J.). Modified nucleotides have successfully been used to synthesize multiply labelled oligonucleotides (Misra et al. (2004) Bioconjug. Chem. 15(3):638-646 and Brumbaugh et al. (1988) PNAS 85:5610-5614).

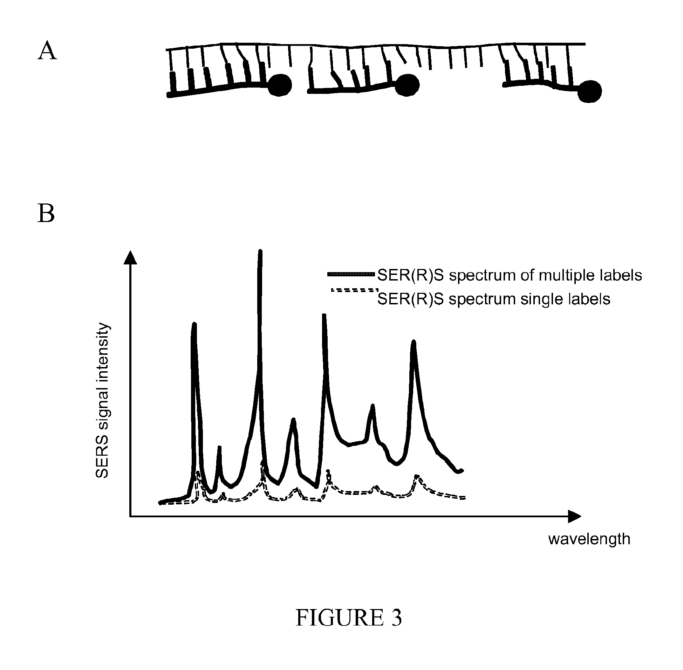

In yet alternative embodiments of methods according to this aspect of the invention, the provision of a multiple-labelled amplicon is ensured by hybridizing the amplicon with one or more amplicon-specific probes carrying multiple identical labels. According to the present invention, the resulting hybridized amplicon should comprise at least two labels to ensure signal amplification. Accordingly, where hybridization is carried out with one probe, this probe should comprise at least two labels having identical optical properties. Where hybridization is carried out with two or more probes, each probe comprises one or more labels, such as one or more SE(R)RS labels. The latter embodiment is illustrated in Figure 3.

The probes used in methods of the present invention include standard oligonucleotide probes which are designed to bind specifically to the amplification product of the nucleic acid amplification reaction of the methods of the present invention. Additionally or alternatively, nucleic acid analogs can be used as probes, such as peptide nucleic acids (PNAs), locked nucleic acid (LNAs), or morpholinos (Karkare and Bhatnagar (2006) Appl. Microbiol. Biotechnol. 71(5):575-86). Where two or more labelled probes are used, these are designed such that each probe hybridizes with a different sequence on the amplified nucleic acid molecule. In addition, the probes are selected such that they do not interfere with the binding of any of the other probes selected for hybridizing to the amplified nucleic acid molecule. Optionally, as indicated above, the target sequence of the different probes is selected such that the cumulative optical signal generated by the different labels is optimal. Where the labels used are fluorescent labels the target sequence of the different probes is selected so as to avoid mutual quenching of the different labels.

Methods for generating labelled probes are known to the skilled person and are similar to the methods for generating labelled primers described above.

In the methods according to these embodiments the nature of the nucleic acid amplification step is not critical and carried out prior to the labelling of the amplicon with multiple labels. The different nucleic acid amplification methods known to the skilled person and referred to above are suitable for generating the amplified nucleic acid molecule in the methods according to these embodiments of the invention.

In a second aspect of the invention, methods are provided which allow the detection and/or quantification of one or more analytes in a sample through two or more different signals for each analyte, thereby improving accuracy of detection of the analyte(s). This is generally achieved by directly or indirectly labelling (each of) the analyte(s) with two or more labels having different optical properties, such that, in parallel, different optical signals are generated, each representative of the presence and/or quantity of the analyte in the sample. Most particularly the methods of the invention comprise the use of at least two differentially labelled probes which either bind the analyte directly or a sequence linked to the analyte. In particular embodiments of this aspect of the invention differential labelling is combined with nucleic acid amplification, to ensure sensitive detection. The methods according to this aspect of the invention are particularly developed for detection using optical labels such as SE(R)RS and/or fluorescent labels.

Similar to the methods described above, the most straightforward embodiments of the methods according to this aspect of the invention involve the detection of one or more analytes in a sample by providing each of the analytes with two or more labels having different optical properties to generate, in parallel, different detection signals. This can be ensured by making use of one probe comprising two or more labels with different optical properties. According to particular embodiments however, this is ensured by contacting the analyte with two or more probes each carrying a different optical label. Where the analyte of interest is a nucleic acid molecule, the labelled probes are each directed against a different sequence within the analyte, such that the different probes can bind to the analyte simultaneously. The sequences targeted by the analyte-specific probes can be selected such that the different signals generated upon the binding of the different probes to the analyte do not interfere. More particularly, where the labels are SE(R)RS labels or fluorescent labels, the position of the probes on the analyte is optimized for optimal SE(R)RS/fluorescent detection of each of the labels. Where the analyte is a protein or peptide, the use of differentially labelled probes is also envisaged. For instance, different antibodies (or antigen-binding fragments thereof) each binding to a different epitope of the analyte, and labelled with a label having different optical properties, are used simultaneously to ensure the simultaneous generation of different detection signals.

According to a particular embodiment, methods of the invention involve amplifying the analyte or a nucleic acid sequence linked specifically thereto and ensuring that the amplified nucleic acid sequence comprises at least two different optical labels. These methods combine increased sensitivity (amplification of the signal) with a control on accuracy of detection. The methods according to this embodiment thus encompass measuring the spectrum of two or more optical labels provided on the amplified nucleic acid sequence to determine the presence and/or quantity of the analyte.

In one embodiment, methods of the invention encompass amplifying the analyte, a fragment thereof or a nucleic acid sequence linked thereto and thereby introducing two or more different optical labels into the amplified nucleic acid sequence.

In particular embodiments, methods of the invention comprise the amplification of the analyte, a fragment thereof or a nucleic acid sequence linked thereto in a nucleic acid amplification method using one primer with at least two different optical labels or two or more primers, at least two of which comprise different labels. The resulting amplified nucleic acid, comprises at least two labels with different optical properties, the signal of each of which is indicative of the presence and quantity of the analyte in the sample.

According to particular embodiments of methods according to this aspect of the invention, the analyte is a nucleic acid and the methods include the steps of amplifying a nucleic acid sequence corresponding to at least a fragment of the analyte in a nucleic acid amplification process, thereby ensuring that two or more optically active labels having different optical properties are incorporated into the amplified nucleic acid molecule and detecting the optical spectrum of each of the different optically active labels incorporated into the amplified nucleic acid molecule.

In other embodiments of methods the invention, the step of providing an amplified nucleic acid molecule which is labelled with at least two different labels is ensured by the use, in the nucleic acid amplification process, of nucleotides carrying one or more optically active labels which have different optical properties. A single nucleotide, e.g., A, C, T, U, or G, carrying one or more labels with different optical properties or several different nucleotides carrying one or more labels with different optical properties can be used in the process. In particular embodiments the optically active labels are labels compatible with SE(R)RS-based detection and generate a different SE(R)RS spectrum.

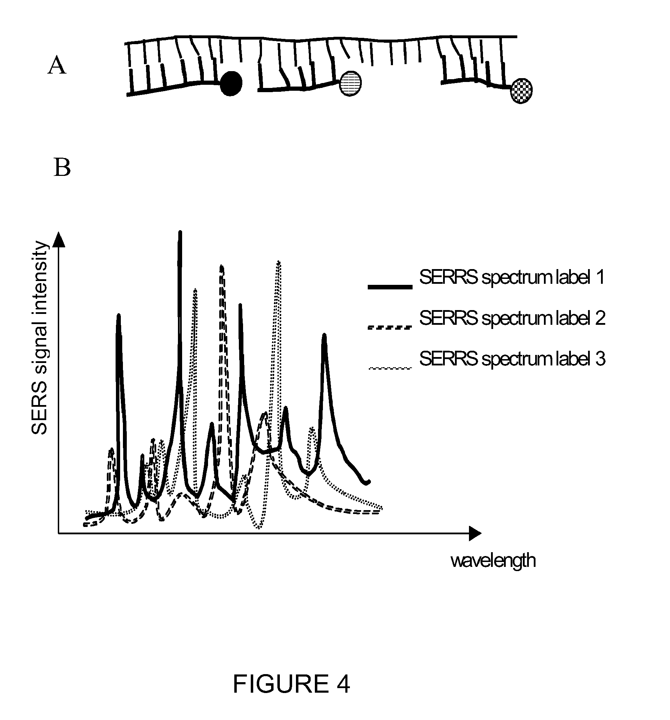

In yet alternative embodiments of methods according to this aspect of the invention, the provision of an amplicon which is labelled by at least two different optically active labels is ensured by hybridizing the amplicon with one or more amplicon-specific probes carrying labels with different optical properties. According to an aspect of the present invention, the resulting hybridized amplicon should comprise at least two optically active labels with different optical properties to allow detection of the analyte based on at least two different signals indicative of the presence of the analyte. Accordingly, similar to the methods described above, where hybridization is carried out with one probe, this probe should comprise at least two different labels. However, according to more particular embodiments, hybridization is carried out with two or more probes each comprising one or more labels whereby at least two of the labels present on these two or more probes differ in their optical properties. The latter embodiment is illustrated in Figure 4.

The resulting amplified nucleic acid carries at least two optically active labels with different optical properties, thereby creating the possibility of detecting two different detection signals and optionally using one of those signals as a control. The reagents used in the methods according to this aspect of the invention, i.e., the primers comprising two or more different optically active labels, the differentially labelled nucleotides, and the probes comprising different optically active labels can be synthesized using the methods described above. In particular embodiments of the above methods the optically active labels are labels compatible with SE(R)RS-based detection which generate a different SE(R)RS spectrum.

Different combinations of the above-described methods are envisaged which can further increase the sensitivity of detection, by a further amplification of the number of labels incorporated corresponding to the presence of one analyte. More particularly it is envisaged that the methods for detecting and/or quantifying one or more analytes in a sample with improved sensitivity described above can be combined with the methods described above which ensure increased accuracy of detection. Practically, this encompasses providing each analyte with both two or more labels having identical optical properties and two or more labels having different optical properties. More particularly this encompasses the use of two or more probes which are labelled with labels having identical optical properties and two or more probes which are labelled with labels having different optical properties.

In more particular embodiments methods combining both aspects of the invention encompass amplifying the analyte, a fragment thereof or a nucleic acid linked to the analyte and providing an amplified nucleic acid molecule which comprises both multiple identical optically active labels (to increase sensitivity of detection) and at least two different optically active labels (to allow a control on accuracy of detection).

In particular embodiments, methods of the present invention involve the amplification of a nucleic acid sequence which is either part of or linked to the analyte. Methods of nucleic acid amplification include, but are not limited to, PCR, Rolling circle amplification (RCA), RT-PCR, NASBA, linear amplification, which methods are extensively described in the art. Suitable conditions for the application of these methods can be easily determined by one of skill in the art following well-established protocols detailed in, *e.g.,* "Molecular Cloning: A Laboratory Manual" by J. Sambrook and D. Russell (2000) Cold Spring Harbour Laboratory Press Ed.

Methods of the present invention involve the detection of two or more optical labels, such as e.g. SE(R)RS labels which are either bound on the analyte directly or incorporated into or provided onto an amplified nucleic acid molecule. Factors that will affect detection are the proximity of the labels to each other and the their accessibility for detection. Moreover, where the labels are SE(R)RS labels the proximity of the labels to the SE(R)RS surface will influence detection (see below). Accordingly, optimally, the location targeted by the different probes used in the context of the present invention or the length of nucleic acid amplified during the nucleic acid amplification process (and/or the position of the labels therein or the labelled probes hybridizing therewith) is optimised for gain in sensitivity of detection, more particularly for gain of sensitivity of SE(R)RS detection.

The amplification of the nucleic acid molecule (corresponding to the analyte, a fragment thereof or a sequence linked to the analyte) envisioned in particular embodiments of the methods of the invention can take place with a mixture, in various proportions, of labelled and unlabelled nucleotides and/or primers. The determination of the optimal conditions of amplification is well within the skills of one skilled in the art, as illustrated in, *e.g.,* Boleda et al., Biotechniques. (1996) 1:134-40.

Methods of the present invention involve the detection and/or quantification of an analyte. The methods of the present invention have been particularly developed for the detection of nucleic acids such as, but not limited to genomic DNA, viral DNA, bacterial DNA, fungal DNA, mammalian DNA, or DNA fragments. The methods of the present invention are similarly applicable to analytes which are RNA such as viral RNA, mRNA, rRNA. The analyte can also be cDNA, oligonucleotides, or synthetic DNA, RNA, PNA (*see*, *e.g.*, Nielsen et al. (1991) Science, 254: 1497-1500), synthetic oligonucleotides, modified oligonucleotides or other nucleic acid analogs. The analyte may comprise single-stranded and double-stranded nucleic acids. It may, prior to detection, be subjected to manipulations such as digestion with restriction enzymes, copying by means of nucleic acid polymerases, shearing or sonication thus allowing fragmentation to occur.

However, as detailed above, subject to specific modifications depending on the analyte, the methods of the invention are equally applicable for the detection of alternative analytes. A non-exhaustive list of analytes includes a protein, polypeptide, peptide, amino acid, nucleic acid, oligonucleotide, nucleotide, nucleoside, carbohydrate, polysaccharide, lipopolysaccharide, glycoprotein, lipoprotein, nucleoproteins, lipid, hormone, steroid, growth factor, cytokine, neurotransmitter, receptor, enzyme, antigen, allergen, antibody, metabolite, cofactor, nutrient, toxin, poison, drug, biowarfare agent, biohazardous agent, infectious agent, prion, vitamin, immunoglobulins, albumin, haemoglobin, coagulation factor, interleukin, interferon, cytokine, a peptide comprising a tumor-specific epitope and an antibody to any of the above substances. An analyte may comprise one or more complex aggregates such as but not limited to a virus, bacterium, fungus, microorganism such as *Salmonella, Streptococcus, Legionella, E. coli, Giardia, Cryptosporidium, Rickettsia,* spore, mold, yeast, algae, amoebae, dinoflagellate, unicellular organism, pathogen or cell, and cell-surface molecules, fragments, portions, components, products, small organic molecules, nucleic acids and oligonucleotides, and metabolites of microorganisms.

The detection of analytes which are not nucleic acid molecules according to the methods of the present invention is optionally carried out by providing the multiple labels directly on the analyte using analyte-specific probes. Examples of analyte specific probes for non-nucleic acid molecules include but are not limited to probes which bind based on antigen-antibody interaction, or an interaction based on carbohydrate-lectin, ligand-receptor, coenzyme-enzyme, enzyme inhibitors-enzyme interactions etc.

According to particular embodiments of methods of the invention, the detection involves an amplification step, so as to ensure increased sensitivity of detection. For the detection of analytes which are not nucleic acid molecules or which are not nucleic acid molecules which can be amplified using a nucleic acid amplification process, these embodiments of the methods of the invention, in addition to the steps described above for the detection of a nucleic acid, encompass the linking of a nucleic acid molecule to the analyte. Depending on the nature of the analyte, the linking of a nucleic acid to the analyte can be ensured in different ways. According to particular embodiments this is ensured by a capture molecule capable of binding specifically to the analyte, to which a nucleic acid molecule has been linked. Depending on the analyte, the binding of the analyte is based on complementary nucleotide sequences, carbohydrate-lectin, complementary peptide sequences, ligand-receptor, coenzyme-enzyme, enzyme inhibitors-enzyme, antigen-antibody binding, etc...

According to a particular embodiment, the analyte is captured by an antibody to which a nucleic acid molecule has been linked and which can be amplified by PCR. This technique, referred to as Immuno-PCR (Sano et al., (1992) Science 258 (5079): 120-122; Banin et al. (2004) Clin. Chem. 50: 1932-1934) allows the ultrasensitive analysis of proteins and other antigens. Similarly, capture antibodies can also be conjugated with double-stranded oligonucleotides which contain the T7 promoter, whereafter the oligonucleotide is amplified using RNA polymerase (as described in Zhang et al., 2001, PNAS 98: 5497, 5502). These techniques combine the well-established ELISA methodology with the signal amplification power of the polymerases. As such these lead to an about 100 to 10,000-fold gain in sensitivity compared to conventional ELISA. In combination with the use of multiple labels according to the present invention, sensitivity is further increased.

Antibody molecules suitable for use (either as analyte-specific probes or as capture molecules) in the methods of the invention include monoclonal antibodies and fragments thereof, e.g., F(ab) and F(ab')2 portions, Fv, and single-chain antibodies, single variable domains, nanobodies and any synthetic antigen-binding fragments. Particular fragments can be generated by treating a classical antibody with an enzyme such as pepsin. The preparation, identification, and, more generally, the use of antibodies is well known in the art and has been extensively described in, e.g., "Immunological Protocols" Robert Bums in "Methods in Molecular Biology", Ed. Humana Press (2005)

The nucleic acid molecules are typically bound to the capture molecules capable of binding to the analyte by way of a linker. More particularly, both the capture molecule and the nucleic acid molecule are biotinylated and these are then linked through a streptavidin molecule. Other linkers that can be used to link a nucleic acid to a capture molecule include covalent bindings and linker molecules such as succinimidyl-4-[N-maleimidomethyl]-cyclohexane-1-carboxylate (SMCC).

In particular embodiments, methods of the present invention involve surface-enhanced (resonance) Raman spectroscopy SE(R)RS, which typically allows for sensitive quantitative detection of analytes in a wide range of concentrations.

SE(R)RS labels suitable for use in the methods of the present invention include, but are not limited to, fluorescein dyes, such as 5- (and 6-) carboxy-4',5'-dichloro-2',7'-dimethoxy fluorescein, 5-carboxy-2',4',5',7'-tetrachlorofluorescein and 5-carboxyfluorescein; rhodamine dyes such as 5- (and 6-) carboxy rhodamine, 6-carboxytetramethyl rhodamine and 6-carboxyrhodamine X, phthalocyanines such as methyl, nitrosyl, sulphonyl and amino phthalocyanines, azo dyes such as those listed in U.S. Pat. No. 6,127,120, azomethines, cyanines and xanthines such as the methyl, nitro, sulphano and amino derivatives, and succinylfluoresceins. Certain naturally occurring compounds such as amino acids (e.g., arginine, methionine, cysteine), nucleic acid bases (e.g., adenine, or guanine) or chemical derivatives thereof can also generate a Raman signal.

It is noted that the choice of the label can be influenced by factors such as the resonance frequency of the label, the resonance frequency of other molecules present in the sample, etc... SE(R)RS-reactive labels of use for detecting biomolecules are described in the art such as in U.S. Pat. Nos. U.S. 5,306,403; U.S. 6,002,471; and U.S. 6,174,677.

Detection by surface-enhanced spectroscopies such as surface-enhanced (resonance) Raman spectroscopy (SE(R)RS) is based on the strong enhancement of Raman scattering observed for analytes adsorbed onto an appropriate "SE(R)RS-active surface". Typically, the surface is a noble (Au, Ag, Cu) or alkali (Li, Na, K) metal surface. Also appropriate for SE(R)RS detection are nanoparticles of gold, silver, copper and certain other metals which are provided as colloids.

In particular embodiments of the methods of the present invention the SE(R)RS signal is further enhanced by the treatment of the metal surface or nanoparticles with chemicals such as sodium chloride and lithium chloride.

In particular embodiments, methods of the invention involve the use of labelled primers and probes which are bound to or hybridize with the analyte or hybridize with/ are incorporated into an optionally amplified nucleic acid. Depending on the set-up of the assay, non-bound labelled probes and/or labelled primers which have not been incorporated can interfere with detection. Accordingly, the methods of the invention optionally comprise, the removal of unbound or un-incorporated labelled probe or primer. The latter step ensures that the intensity of signal of the labelled probe detected is directly correlated with the amount of analyte. Particular embodiments of the methods of the present invention include a step corresponding to a physical separation of the unbound/non-incorporated labelled probe or primer from the labelled amplified nucleic acid within the sample. This can be achieved by providing a tag to the labelled primers or probes or by providing a secondary probe capable of binding to the amplified nucleic acid comprising a tag, which can be subjected to physical and/or chemical forces. Typical examples of such tags include magnetic beads (which can be subjected to magnetic forces), glass or polystyrene beads (which can be captured and moved by a light beam, Smith et al. (1995), Science 271:795), or charged groups (which can be subjected to electrokinetic forces (Chou et al. (1999), PNAS 96:11). Optionally, the tag can be directly incorporated into the amplified nucleic acid or amplicon.

The present invention relates to improved methods for the detection and/or quantification of an analyte, either in pure form or as an analyte in a sample. While the methods described herein will generally refer to 'an analyte' it is equally envisaged that the methods of the present invention can be applied where several analytes are being detected or quantified simultaneously, using different analyte-specific labels, such as differentially labelled primers or probes. Accordingly, one of the advantages of SE(R)RS is that it allows the simultaneous detection of different labels, which are used in the aspect of the present invention involving detecting the analyte with increased accuracy can additionally or alternatively be applied for "multiplexing". In this regard it will be understood by the skilled person that the methods of the present invention can be applied to the detection of different analytes within one sample, whereby each of the analytes is detected with multiple probes with the same (and/or different identical properties).

The present invention allows for the improvement of the sensitivity and accuracy of the detection and/or quantification of one or more analytes in a sample. The term "sample" is used in a broad sense herein and is intended to include a wide range of biological materials as well as compositions derived or extracted from such biological materials. The sample may be any suitable preparation in which the analyte is to be detected. The sample may comprise, for instance, a body tissue or fluid such as but not limited to blood (including plasma and platelet fractions), spinal fluid, mucus, sputum, saliva, semen, stool or urine or any fraction thereof. Exemplary samples include whole blood, red blood cells, white blood cells, buffy coat, hair, nails and cuticle material, swabs, including but not limited to buccal swabs, throat swabs, vaginal swabs, urethral swabs, cervical swabs, rectal swabs, lesion swabs, abscess swabs, nasopharyngeal swabs, nasal swabs and the like, lymphatic fluid, amniotic fluid, cerebrospinal fluid, peritoneal effusions, pleural effusions, fluid from cysts, synovial fluid, vitreous humor, aqueous humor, bursa fluid, eye washes, eye aspirates, plasma, serum, pulmonary lavage, lung aspirates, biopsy material of any tissue in the body. The skilled artisan will appreciate that lysates, extracts, or material obtained from any of the above exemplary biological samples are also considered as samples. Tissue culture cells, including explanted material, primary cells, secondary cell lines, and the like, as well as lysates, extracts, supernatants or materials obtained from any cells, tissues or organs, are also within the meaning of the term biological sample as used herein. Samples comprising microorganisms and viruses are also envisaged in the context of analyte detection using the methods of the invention. Materials obtained from forensic settings are also within the intended meaning of the term sample. Samples may also comprise foodstuffs and beverages, water suspected of contamination, etc. These lists are not intended to be exhaustive.

In a particular embodiment of the invention, the sample is pre-treated to facilitate the detection of the sample with the detection method. For instance, typically a pretreatment of the sample resulting in a semi-isolation or isolation of the analyte or ensuring the amplification of the analyte is envisaged. Many methods and kits are available for pretreating samples of various types.

Particular embodiments of the methods of the invention relate to methods of detection based on SE(R)RS, and typically involve operating at the resonant frequency of the SE(R)RS labels used, which gives increased sensitivity. The light source used to generate the Raman spectrum for this purpose is a coherent light source, e.g., a laser, tuned substantially to the maximum absorption frequency of the label being used. This frequency may shift slightly on association of the label with the SE(R)RS-active surface and based on the binding to or incorporation in the amplicon, but the skilled person will be well able to tune the light source to accommodate this. The light source may be tuned to a frequency in the absorption band, or near to the label's absorption maximum, or to a frequency at or near that of a secondary peak in the label's absorption spectrum. SE(R)RS may alternatively involve operating at, or close to, the resonant frequency of the plasmons on the active surface of the metal colloids. As stated above, the choice of the optimal operating frequency can easily be made by one of ordinary skills in the art based on the metal(s) and label(s) chosen.

In SE(R)RS detection typically the fingerprint spectrum is measured in order to identify each label. However, if the different labels used each have a unique spectral line, then it can be sufficient to detect signal intensity at a chosen spectral line frequency or frequencies. Accordingly, where the methods of the invention encompass detecting the detection signal of the labels used, this corresponds to detection of the entire spectrum or of a signal at a specific frequency or at a discrete number of frequencies. Where different labels with different optical properties are used this encompasses detecting the entire spectrum of each label or a signal at a specific frequency for each label (which is either the same or a different frequency for the different labels).

Typically, the detection step in the SE(R)RS based detection methods of the present invention are carried out using incident light from a laser, having a frequency in the visible spectrum. The exact frequency chosen will depend on the label, surface and analyte. Frequencies in the red and/or green area of the visible spectrum tend, on the whole, to give rise to better surface enhancement effects. However, it is possible to envisage situations in which other frequencies, for instance in the ultraviolet, the near-infrared or infrared ranges, might be used. The selection and, if necessary, tuning of an appropriate light source, with an appropriate frequency and power, will be well within the capabilities of one of ordinary skill in the art, particularly on referring to the available SE(R)RS literature.

Excitation sources for use in SE(R)RS-based detection methods include, but are not limited to, nitrogen lasers, helium-cadmium lasers, argon ion lasers, krypton ion lasers, etc... Multiple lasers can provide a wide choice of excitation lines which is critical for resonance Raman spectroscopy. The laser power, or excitation power, can be varied depending, e.g., on the properties of the label used.

The excitation beam may be spectrally purified with a bandpass filter and may be focused on a substrate using an objective lens. The objective lens may be used to both excite the sample and to collect the Raman signal, by using a holographic beam splitter to produce a right-angle geometry for the excitation beam and the emitted Raman signal. The intensity of the Raman signal needs to be measured against an intense background from the excitation beam. The background is primarily Rayleigh scattered light and specular reflection, which can be selectively removed with high efficiency optical filters. For example, a holographic notch filter may be used to reduce Rayleigh scattered radiation.

Several objective lenses can be used in the context of the invention. According to a specific embodiment, the objective lens is a 5x, 10x, 20x, 50x or a 100x objective lens. In most set ups, the nature of the objective lens influences the excitation volume, since the focal distance, numerical aperture and working distance determine the volume irradiated. Accordingly, where objectives of 5x, 10x, and 50x are used, the excitation volume typically decreases from 5x to 50x, accordingly.

The surface-enhanced Raman emission signal may be detected by a Raman detector. A variety of detection units of potential use in Raman spectroscopy are known in the art and any known Raman detection unit may be used. An example of a Raman detection unit is disclosed e.g. in U.S. Pat. No. 6,002,471. Other types of detectors may be used, such as a charge-coupled device (CCD), with a red-enhanced intensified charge-coupled device (RE-ICCD), a silicon photodiode, or photomultiplier tubes arranged either singly or in series for cascade amplification of the signal. Photon counting electronics can be used for sensitive detection. The choice of detector will largely depend on the sensitivity of detection required to carry out a particular assay. Several devices are suitable for collecting SE(R)RS signals, including wavelength selective mirrors, holographic optical elements for scattered light detection and fibre-optic waveguides.

The apparatus for obtaining and/or analysing a SE(R)RS spectrum may include some form of data processor such as a computer. Once the SE(R)RS signal has been captured by an appropriate detector, its frequency and intensity data will typically be passed on to a computer for analysis. Either the fingerprint Raman spectrum will be compared to reference spectra for identification of the detected Raman active compound or the signal intensity at the measured frequencies will be used to calculate the amount of Raman active compound detected. Therefore, the detection can be qualitative, semi-quantitative or quantitative, when the various spectra obtained as described are compared with an internal standard chosen appropriately, as is well known by a person skilled in the art.

The methods according to these particular embodiments of the invention involving SE(R)RS-based detection not only provide SE(R)RS measurements with a high signal strength. The present invention provides methods allowing detection with an improved (i.e., lower) limit of detection (Lod) when compared to known methods.

A further aspect of the invention provides kits for performing the methods of the present invention. Kits may be conveniently assembled for use in conjunction with the methods of the present invention. The kits contain appropriate specific primers and/or probes for detecting analytes within a sample. Moreover, kits may include standard reagents, such as positive and negative controls, or pre-measured analytes for dose response curves. Such kits optionally include buffers and blocking solutions that are often used for intermediate assay steps, which reduce non-specific binding of primers and/or probes to analytes, as is well known by the person skilled in the art. Such kits optionally also include appropriate reagents to perform the amplification of nucleic acid molecules according to the methods of the present invention described herein. Kits of the invention comprise combinations of primers or probe which, when hybridizing with the target, result in a multiple-labelled target. In particular embodiments, combinations of at least 3, more particularly 4 to 10 primers or probes are envisaged each comprising one or more optically active labels. In further embodiments, combinations of at least 2, more particularly 3 to 10 primers or probes are provided each comprising two or more optically active labels. In particular embodiments, the labels are compatible with SE(R)RS-based detection. In particular embodiments at least two of the labels provided on each primer or probe have identical optical properties. In yet other particular embodiments of the present invention, at least two of the labels provided on the primers and probes in the kits of the present invention have different optical properties.

### EXAMPLES

### Example 1: High sensitivity detection of HIV using multiple labelled primers

The analyte to be detected and/or quantified is the *gag* gene for the detection of HIV.

Complementary primers are designed to specifically amplify a sequence within the *gag* gene. The primers each carry two or more identical SE(R)RS labels. These are obtained by replacement of thymidine with 5-C6-Amino-2'-deoxythymidine during the synthesis of the oligonucleotides (IBA GmbH, Göttingen). After the synthesis is completed, a labelling reaction is performed with amine-reactive carboxyrhodamine 6G succinimidyl ester (Invitrogen) to incorporate carboxyrhodamine 6G SE(R)RS labels. Before use, the labelled oligonucleotide is purified by HPLC. The amplification of the *gag* gene in the sample is carried out using standard PCR methods and the resulting amplicon is detected using SE(R)RS.

As illustrated in FIG.1, the resulting amplicon carries the SE(R)RS labels which are incorporated into the primers. This increases the strength of the amplicon's signal and its interaction with the metal surface used in SE(R)RS. Such an increased interaction leads to a better reproducibility of the signal.

### Example 2: Detection of HIV with increased sensitivity using multiple labelled probes

The analyte to be detected and/or quantified is the *gag* gene for the detection of HIV.

Complementary primers are designed to specifically amplify a sequence within the *gag* gene. In a second step the amplicon is hybridized with three different probes each complementary to a different sequence within the amplicon. These probes are 5' labelled with the same SE(R)RS label, TAMRA, via incorporation of a 5' TAMRA phosphoramidite residue (emp Biotech GmbH, Berlin) during the synthesis of the oligonucleotide.

As illustrated in FIG.3A, the resulting amplicon carries three copies of the TAMRA SE(R)RS label. This increases the strength of the amplicon's signal and its interaction with the metal surface used in SE(R)RS. Such an increased interaction leads to a better reproducibility of the signal (Figure 3B)

### Example 3: Detection of HIV infection with increased accuracy

The analyte to be detected and/or quantified is the *gag* gene for the detection of HIV.

Complementary primers are designed to specifically amplify a sequence within the *gag* gene. The resulting amplicon is hybridized with three different probes each complementary to a different sequence within the *gag* amplicon. The probes each carry a different SE(R)RS label. These are obtained by coupling Carboxyrhodamine 6G to the 5' end of a first probe and TAMRA to the 5' end of a second probe during the synthesis of the oligonucleotides. The third *gag* amplicon-specific probe is labelled at the 5'-terminus with Cy3 dye, by incorporation of a Cy3 amidite (commercially available from GE Healthcare).

As illustrated in FIG. 4A, the resulting amplicon carries three different SE(R)RS labels. The amplicon is then put into contact with a metallic surface to enhance the Raman signals and the signals are then detected and analysed.

As shown in FIG 4B, the different SE(R)RS signals can be analysed separately and provide, in one reading, different signals for the detection of the same analyte. This allows a control over the accuracy of detection.

## Claims

1. A method for sensitive detection of an analyte in a sample, comprising the steps of providing on a nucleic acid molecule, which is the analyte, a fragment thereof, or a nucleic acid sequence linked to the analyte, two or more optically active labels having the same optical properties and detecting the optical signal of said optically active labels provided on said nucleic acid molecule as a measure for the amount of analyte in the sample.

2. The method of claim 1, which comprises amplifying the nucleic acid molecule.

3. The method of claim 2, which comprises amplifying said nucleic acid molecule using primers each of which comprises one or more labels having the same optical properties.

4. The method of claim 2, which comprises amplifying said nucleic acid molecule using nucleotides of which at least a percentage comprise the same optical label.

5. The method of claim 1, wherein the two or more labels having the same optical properties are SE(R)RS labels having the same optical properties.

6. The method of claim 1 or 2, which comprises allowing the nucleic acid molecule to hybridize to two or more nucleic acid probes, each of said nucleic acid probes being complementary to a different sequence of said nucleic acid molecule, whereby the two or more nucleic acid probes together comprise at least two labels having the same optical properties.

7. The method according to claim 6, wherein each nucleic acid probe comprises one label.

8. The method according to claim 6, wherein each nucleic acid probe comprises more than one label.

9. The method according to claim 1 or 2, wherein the analyte is DNA, RNA, or PNA.

10. The method according to claim 2, wherein the nucleic acid amplification process comprises PCR, RCA, NASBA, or linear amplification.

11. The method according to claim 2, wherein the optically active labels are SE(R)RS labels and wherein the length of the fragment of said nucleic acid amplified during the nucleic acid amplification process is optimised for gain in sensitivity of SE(R)RS.

12. The method according to claim 1 or 2, wherein the analyte comprises a non-nucleic acid molecule and the method further comprises the step of binding, directly or indirectly, a nucleic acid molecule to said non-nucleic acid molecule.

13. The method according to claim 12, wherein the non-nucleic acid molecule is a peptide, a polypeptide or a protein.

14. The method according to claim 13, wherein the non-nucleic acid molecule is bound to the nucleic acid molecule directly or indirectly through an antibody.

15. The method of claim 1 or 2, further comprising the step of correlating the intensity of the detected optical signal of the labels on said nucleic acid to the amount of analyte in the sample.

16. A method to increase precision of SE(R)RS-based detection of an analyte in a sample, comprising the steps of providing on a nucleic acid molecule, which is the analyte, a fragment thereof, or a nucleic acid sequence linked to the analyte, two or more labels having different optical properties and detecting the signal of each of said labels provided on said nucleic acid molecule.

17. The method of claim 16, wherein the nucleic acid molecule is subjected to a nucleic acid amplification process.

18. The method of claim 17, which comprises amplifying said nucleic acid molecule with nucleotides comprising at least two labels having different optical properties.

19. The method of claim 16 or 17, which comprises allowing the nucleic acid molecule to hybridise with two or more nucleic acid probes, each of said nucleic acid probes being complementary to a different sequence of said nucleic acid molecule and each probe comprising at least one label having different optical properties.

20. The method according to claim 16 or 17, wherein the analyte comprises a non-nucleic acid molecule and wherein said method further comprises the step of binding, directly or indirectly, a nucleic acid molecule to said non-nucleic acid molecule.

21. The method according to claim 20, wherein the non-nucleic acid molecule is a peptide, a polypeptide or a protein.

22. The method according to claim 20, wherein the non-nucleic acid molecule is bound to the nucleic acid molecule directly or indirectly through an antibody.

23. The method according to claim 16 or 17, wherein the analyte is DNA, RNA, or PNA.

24. The method according to claim 17, wherein the nucleic acid amplification process comprises PCR, RCA, NASBA, or linear amplification.

25. The method of claim 16 or 17, further comprising the step of correlating the intensity of the signal of at least one of the labels provided on the nucleic acid to the amount of analyte in the sample and comparing the intensity of the signal of the different labels provided on the nucleic acid to determine the accuracy of detection.

26. The method according to claims 16 or 17, wherein said two or more labels having different optical properties, are SE(R)RS labels having different optical properties.

27. A kit for use in the detection of an analyte in a sample, comprising at least two primers or probes specific for the analyte, each carrying two or more labels.

28. A kit for use in the detection of an analyte in a sample, comprising at least three primers or probes specific for the analyte, each carrying one or more labels.

29. The kit of claim 27 or 28, wherein said labels are SE(R)RS labels having the same optical properties.

30. The kit of claim 27 or 28, wherein said labels are SE(R)RS labels having different optical properties.
